(19) Europäisches Patentamt
European Patent Office
Office européen des brevets

(11) Publication number: 0 091 128
B1

(12) **EUROPEAN PATENT SPECIFICATION**

(45) Date of publication of patent specification: **29.07.87**

(51) Int. Cl.$^4$: **A 61 L 15/01**, C 08 F 283/00, C 08 J 5/18

(21) Application number: **83103356.8**

(22) Date of filing: **06.04.83**

(54) **Synthetic wound covering.**

(30) Priority: **06.04.82 IL 65438**

(43) Date of publication of application:
**12.10.83 Bulletin 83/41**

(45) Publication of the grant of the patent:
**29.07.87 Bulletin 87/31**

(84) Designated Contracting States:
**CH DE FR GB IT LI NL SE**

(56) References cited:
**EP-A-0 046 071**
**FR-A-2 187 849**
**GB-A-1 419 669**
**GB-A-2 035 350**
**US-A-3 657 060**

**JOURNAL OF APPLIED POLYMER SCIENCE, vol. 22, no. 1, January 1978, pages 643-664, John Wiley & Sons, Inc. New York, US, B.D. RATNER et al.: "Radiation-grafted hydrogels for biomaterial applications as studied by the ESCA technique"**

(73) Proprietor: **THE STATE of ISRAEL Atomic Energy Commission Soreq Nuclear Research Center**
**Nahal Soreq**
**Yavne 70 600 (IL)**

(73) Proprietor: **Ben-Hur, Nahum**
**8 Bialik Street**
**Jerusalem 96 221 (IL)**

(72) Inventor: **Behar, David**
**4 Habanai Street**
**Jerusalem 96 264 (IL)**
Inventor: **Juszynski, Marta**
**43/9 Epstein Street**
**Rehovot (IL)**
Inventor: **Rajbenbach, Arie L.**
**11 Herzog Street**
**Givatayim 53 600 (IL)**
Inventor: **Ben-Hur, Nahum**
**8 Bialik Street**
**Jerusalem 96 221 (IL)**

(74) Representative: **Wuesthoff, Franz, Dr.-Ing. et al**
**Patentanwälte Wuesthoff -v. Pechmann-Behrens-Goetz Schweigerstrasse 2**
**D-8000 München 90 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 091 128 B1

Courier Press, Leamington Spa, England.

**Description**

The present invention relates to water permeable films for use as skin substitute, i.e. a temporary burn covering, wound dressing and the like which remains on the wound for the duration of the healing process and peels off once the wound is sealed by the patient's own skin. More specifically the invention relates to synthetic skin substitutes which in the following specification and claims will be referred to as "artificial skin".

A skin substitute should have the following properties;

(i) it must have optimal water permeability which will prevent either dessication of the wound or fluid accumulation under the covering;

(ii) it must prevent microbial invasion from the environment;

(iii) it must have no antigenic properties;

(iv) it must be an elastic-plastic film to facilitate intimate cover of the wound so that it will conform well to all possible contours of the human body;

(v) it must be capable of adhering well to the wound and of being readily removable without causing any damage to the tissue beneath the cover;

(vi) it must prevent scar formation;

(vii) it must be readily available, amenable to storage and inexpensive to produce.

To date no artificial material is known that meets all these requirements and consequently the presently employed methods for covering burns and similar wounds comprise the use of skin substitutes of natural origin, either allograft, a skin from another person, mainly cadaver skin, or xenograft, a skin from another animal, mainly porcine skin.

Although a great deal of effort has been directed during the last years towards the development of artificial skins, no successful commercial replacement for biological skin in the treatment of major skin wounds has been reported. Several synthetic polymeric membranes have been used as temporary covers for burns and open wounds of which the commercial product known by the name Op-site is the most common in use. The main drawback of this product is its low permeability to water which causes a fluid buildup underneath the covering.

The biological skin substitutes have some advantages, but their use is limited. Allograft for example, gives rise to problems of antigenicity, bacterial contamination, complications in handling and limited availability. Pig skin xenografts have a tendency of being digested by the collagenase present in the wound which subsequently might cause infections.

The present state of the art may thus be summed up in that while biological skin substitutes are of limited use no adequate artificial skin substitutes are commercially available yet and it is thus evident that there existed a long-felt want for the development of artificial skins for burn covering, wound dressing, etc. and it is the object of the present invention to provide such artificial skins.

In accordance with the present invention there is provided an artificial skin as a synthetic wound covering consisting of a thin, tough, elastic hydrophilic membrane whose thickness does not exceed 60 µm having a water permeability of 2,400—8,000 g/m²/24h at 37°C and 60% relative humidity being a product obtained by grafting a hydrophilic monomer to a thin body of a polyester polyurethane having a recurring unit of the formula

$$—[(OR_1COOR_2CO)_nOR_1OCONH—C_6H_4CH_2C_6H_4—NHCO]—$$

in which $R_1$ and $R_2$ are each a group $—(CH_2)_x—$ where x is an integer of 1 to 8, and n has an average value of 2.5 to 20, which polyester polyurethane has a modulus of elasticity below 1034 N/cm² (1500 PSI) and elongating at break above 500%.

Preferably an artificial skin according to the invention is not thinner than 10 µm.

Polyurethane is the accepted term for a variety of elastomers obtained by copolymerization of isocyanate with polyols. The polyols may be monomeric or polymeric such as polyesters, polyester amides or polyethers while the isocyanates are usually employed in monomeric form. Generally an isocyanate can be described by the formula RNCO where R is an organic radical. Thus for the production of a polyurethane both the polyol and the isocyanate components can be selected from among a large variety of products and it is thus evident that the term "polyurethanes" covers a very large number of different elastomers with different properties. None of the known commercial polyurethanes have the properties which would render them suitable as artificial skins and it was surprising to find in accordance with the invention that certain polyester polyurethanes can be converted into artificial skins that have all the desired properties, by grafting thereon a hydrophilic polymer. As a result of the grafting a new homogenous membrane is obtained where the graft penetrates through the whole thickness of the polyurethane. The resulting membrane can be viewed as a new material whose properties significantly differ from the original polyurethane.

The suitability or otherwise of a given polyester polyurethane for the purposes of the present invention can easily be determined by a test tube experiment in which a thin polyester polyurethane film is immersed in an aqueous solution of a hydrophilic monomer and a salt of a transition metal whose ion is capable of serving as an inhibitor for the homopolymerization of the monomer. The solution is then subjected to

conditions which cause the formation of free radicals, for example irradiation by $^{60}Co$. After a certain time, e.g. 2—4 hours the film is removed from the solution and its water permeability is determined at 37°C and 60% R. H. According to ASTM E96-66 (Procedure B. W. Inverted Water Method). If the water permeability of the product under these conditions is between 2,400 g/m²/24h to 8,000 g/m²/24h and if the surface of said product is wettable then the starting material is suitable while if the water permeability remains below 2,400 g/m²/24h and the surface is not wettable, it is not.

Some preferred polyester polyurethanes applicable in accordance with the invention and whose performance meets with the requirements as determined by a test tube experiment as set out above, are polyester polyurethanes resulting from copolymerization of butane-diol, adipic acid and p,p'-diphenylmethane diisocyanate, where the butanediol and adipic acid are the polyester constituents bearing alcohol end groups which react with the diisocyanate to form the polymer. As mentioned such polymers are characterised by a recurring unit of the formula

$$-\!\!\!+(OR_1OCOR_2CO)_nOR_1OCONH—C_6H_4CH_2C_6H_4—NHCO\!\!\!+\!\!-$$

where $R_1$ and $R_2$ are each a group $—(CH_2)_x—$ where x is an integer from 1 to 8 and the average value of n is 2.5—20.

In accordance with a particular embodiment of the invention $R_1$ and $R_2$ are each $—(CH_2)_4—$. Preferably the average value of n is 2.5 to 8.

The yield of grafting and the properties of the artificial skin produced by the procedure described depend on the value of n and the elastic properties of the polymer used.

The invention also provides a method for producing artificial skins as defined above comprising preparing an aqueous solution of hydrophilic monomer and a salt of a transition metal whose ion is capable of serving as homopolymerization inhibitor, immersing into this solution a thin film of polyurethane of the kind defined above and whose thickness does not exceed 60 μm, subjecting the solution to conditions inducive to free radical graft copolymerization and continuing the reaction until a product of the desired permeability is obtained.

The hydrophilic monomer used for grafting on a polyurethane in accordance with the present invention can be selected from the group of vinyl type monomers such as acrylamide, hydroxyethyl acrylate, hydroxyethyl methacrylate and acrylic acid. The conditions inducive for free radical graft copolymerization may either be a high energy ionization radiation such as a $^{60}Co$ radiation or the addition of an initiator such as a $Ce^{4+}$ salt. Where a $Ce^{4+}$ salt is used as graft polymerization initiator the same salt may fulfil the function of homopolymerization inhibitor.

When graft copolymerization is initiated by ionizing radiation a $Ce^{4+}$ salt may be used as inhibitor. It is thus seen that in the context of the present invention $Ce^{4+}$ salts may serve both as graft copolymerization initiators and as homopolymerization inhibitors.

The range of water permeability stipulated for the artificial skins according to the invention was selected on the basis of the highly increased water flux from a wound surface as compared to a normal undamaged skin. Thus, values ranging from 3,400 to 5,200 g/m²/24h of water flux have been reported, depending on the type of the wound (L. O. Lamke, G. E. Nilsson and H. L. Reithner, Burns *3* 159 (1977)). Therefore, in order to avoid fluid accumulation under the wound cover the water permeability recommended for artificial skins should be much higher than that of natural skin. The average body losses of water through natural skin is of the order of 240 g/m²/24 h only.

Artificial skins according to the invention are relatively unelastic when dry but absorb water and become very elastic when wet. The water absorbence takes place within a few seconds when applying the membrane on a wet surface or immersing it in water. The lack of elasticity of the dry artificial skin renders it easy to store without a backing film, easy to handle for use and it can be applied on a wound without any pretreatment. Moisture from the wound is immediately absorbed by the membrane forming it into a completely elastic material. This compares favourably with, for example, porcine skin which has to be soaked in saline solution prior to application for about 30 minutes.

While artificial skins according to the invention are water permeable they are impermeable to microorganisms, so that dirt of any kind is prevented from passing through such skin.

Once applied to a wound, an artificial skin according to the invention remains elastic as long as the wound is wet and permits a controlled flux of water from the wound, thus preventing either fluid accumulation or dessication of the wound. When the wound heals the artificial skin peels off from the healed part. If a gradual healing occurs gradual peeling off takes place.

The art of chemical radiation grafting of hydrophilic monomers on hydrophobic polymers is well documented but none of the known products is suitable as artificial skin. (See for example A. Chapiro, Radiation Chemistry of Polymeric Systems, Interscience, Academic Press, London 1962). The radiation induced grafting of acrylic monomers onto polyurethane (Tuftane 310) and silicon rubber has been reported (B. D. Ratner, P. K. Weathersby and A. S. Hoffman, J. Appl. Polym. Sci. *22* 643 (1978). A. S. Hoffman and B. D. Ratner Rad. Phys. Chem. *14* 831 (1979)) and in FR—A—218 78 49. In these publications surface grafted films were obtained. Grafting of hydrophiles on Tuftane as reported above resulted in products still having hydrophobic surface character when wet, which makes them less favourable as skin substitutes. Jansen and Ellinghorst (J. Polym. Symp. *66* 465 (1979)) used a preswelling technique to graft

polymerize HEMA into Tuftane 410 polyurethane. Films prepared by us using the later technique were found to have also hydrophobic surface character.

GB—A—2 035 350 discloses among others the production of a bio-compatible film that can be used for the treatment of wounds and burns obtained by copolymerisation of polyetherurethane with acrylic acid. There is no stipulation of any required elasticity of the starting material nor is then any teaching of the required water permeability of the product. The present invention which uses polyester polyurethane as starting material with stipulations for elasticity limits of the starting material and water permeability of the product is well distinguished therefrom.

$Ce^{4+}$ salt was used as initiator for chemical grafting of poly-acrylamide onto polyurethane surface in the development of non-thrombogenic prosthetic devices. (B. D. Halpern, M. K. Akkapeddi and S. L. Ledis, Annual Report PH—43—66—1124—6, PB—230, NIH (1974)).

It is thus evident that not only did the prior art not anticipate the present invention but in actual fact it taught away from it because the teachings thereof did not give the expert any reason to believe that an artificial skin with all the required properties could be made from polyester polyurethane. Consequently the discoveries made in accordance with the present invention were completely surprising.

In the artificial skin according to the invention the graft add-on ranges from 20—400% and preferably from 50—150%. The graft add-on is determined by drying thoroughly the washed product in vacuo at 50°C and then weighing it. The percent of add-on is then calculated as $100\times(w_g-w_i)/w_i$, where $w_i$ and $w_g$ represent the weight of the initial and grafted films, respectively. Notwithstanding the grafted material, an artificial skin according to the invention, is as a rule not appreciably thicker than the polyester polyurethane film used as starting material. However, an increase in the lateral dimension as compared to the original film are observable.

The dramatic changes in the properties of the membrane between dry and wet states are illustrated by looking at the tensile properties of a representative sample of the polyester polyurethane film having 73% grafted poly-acrylamide. The elongation at break of this film in the dried state measured at 50% R. H. is 325% while that of the wet membrane at 95% is 880%. The modulus at 100% elongation of the same dry membrane measured at 50% R. H. is 2184 N/cm$^2$ (3170 PSI) while that of the wet membrane at 95% R. H. is 152 N/cm$^2$ (220 PSI).

Clinical results

Poly-acrylamide grafted polyurethane membranes with add-on ranging from 60 to 160% were used on 20 patients in various clinical situations, such as burns, donor sites and ulcers. The mode of application of the membrane was either in its wet or dry state. In the former case the membrane was immersed in saline solution for a few seconds before use. The membrane changed its appearance and became slightly opaque and pliable when wet but still transparent enough to observe any undergoing process underneath the cover. Upon application there was immediate adherence of the membrane to the denuded area without the need for suturing, as is usually practiced in skin grafting. The membranes remained attached until they were removed, or until a complete healing occurred underneath them. Then they were peeled off by mere washing with water. Any area where the epidermis reached complete recovery the membrane peeled off by itself. Other areas, like ulcers, demonstrated a good granulation tissue underneath it, and in due time, the membrane was peeled off by the treating surgeon and the area was recovered by skin graft.

One of the striking observations was the cessation of pain. Out of 20 patients, in 18 cases very good results were obtained i.e. healing of epidermis under the membrane, or healing of donor area, or preparing the bed for final skin grafting. The membranes are convenient, pliable and self-adherent. There was no sensitivity to them and no infection. In 3 patients, there were hematoma underneath the cover that needed evacuation, but did not interfere with the healing process itself. The membranes remained attached to the treated areas between 3 to 21 days. Most of the wound sizes treated were around 200 cm$^2$.

The invention is further illustrated by the following Examples without being limited thereto. In these Examples the following abbreviations are used:

RH—relative humidity
AAm—acrylamide
HEA—hydroxyethyl acrylate
HEMA—hydroxyethyl methacrylate
AA—acrylic acid.

Example 1

A sheet of polyester polyurethane (30 μm thick) having a recurring unit of the formula

$$-\!\!\!\!+\!(OR_1OCOR_2CO)_nOR_1OCONH—C_6H_4—CH_2—C_6H_4NHCO\!+\!\!\!\!-$$

with average n=6.5. $R_1=R_2=(CH_2)_4$ and having a modulus of elasticity of 362 N/cm$^2$ (525 PSI) and elongation at break of 950%, is immersed in a test tube containing freshly prepared 20% AAm in 20 mM aqueous $Ce(NH_4)_2\,(NO_3)_6$ solution. The $Ce^{4+}$ salt is a homopolymerization inhibitor for the AAm. The test tube and its contents are irradiated at room temperature in the presence of air with a $^{60}Co$ gamma source

for 120 min. receiving a total dose of 2400 Gy (240 krad). The sheet is removed from the test tube and washed with warm water for several hours, then dried in a vacuum oven at 50°C to a constant weight. A graft of 115% was obtained. The water permeability of the grafted membrane was measured and found to be 6800 $g/m^2/24$ h. The amount of water absorbed by the film was about 110% of the total film weight.

Example 2

Repeating the same experimental conditions of Example 1 but decreasing the dose rate so that the total dose absorbed by the sample is 1500 Gy (150 krad) resulted in a membrane having a 106% graft. The water permeability measured was 7600 $g/m^2/24$ h and the water absorbed by the membrane was about 100% of the total film weight.

Example 3

Example 1 was repeated except that the $Ce(NH_4)_2(NO_3)_4$ concentration was increased to 40 mM. The resulted membrane contained 146% graft. The water permeability of the membrane so produced was 7000 $g/m^2/24$ h, and the water absorption about 150% of the total film weight.

Example 4

Example 1 was repeated but the $Ce(NH_4)_2(NO_3)_6$ concentration was increased to 50 mM and the irradiation time increased to 180 min. so that the total dose absorbed by the sample was 3600 Gy (360 krad). The resulted membrane contained 163% graft. The water permeability was 7600 $g/m^2/24$ h and the water absorption was about 150% of the total film weight.

Example 5

Example 1 was repeated except that the dose rate decreased to 13 Gy/min (1.3 krad/min) and irradiation time was 100 min. The resulted membrane contained 82% graft. The water permeability of the membrane so produced was 7200 $g/m^2/24$ h and the water absorption was about 90% of the total film weight.

Example 6

Example 4 was repeated except that the $Ce(NH_4)_2(NO_3)_6$ was replaced by 40 mM $Ce(SO_4)_2$ and made 0.2 N in $H_2SO_4$. The resulted membrane contained 42% graft. The water permeability of the membrane so produced was 6900 $g/m^2/24$ h and the water absorption about 50% of the total film weight.

Example 7

Example 4 was repeated except that the $Ce(NH_4)_2(NO_3)_6$ was replaced by 40 mM $Ce(NH_4)_2(SO_4)_3$. The resulted membrane contained 57% graft. The water permeability of the membrane was 5800 $g/m^2/24$ h and the water absorption about 60% of the total film weight.

Example 8

Example 7 was repeated except that the $Ce(NH_4)_2(SO_4)_3$ was increased to 100 mM. The resulted membrane contained 59% graft. The water permeability of the membrane was 7000 $g/m^2/24$ h and the water absorption about 60% of the total film weight.

Example 9

Example 8 was repeated except that the irradiation time was doubled to 360 min. so that the total dose absorbed by the sample was 7200 Gy (720 krad). The resulted membrane contained 64% graft. The water permeability of the membrane so produced was 6400 $g/m^2/24$ h and the water absorption about 60% of the total film weight.

Example 10

Example 4 was repeated except that the ceric salt was replaced by 20 mM $Cu(NO_3)_2$. The resulted membrane contained 63% graft. The water permeability of the membrane so produced was 7200 $g/m^2/24$ h and the water absorption about 50% of the total film weight.

Example 11

Example 10 was repeated except that the $Cu(NO_3)_2$ was replaced by 100 mM $CuSO_4$. The resulted membrane contained 43% graft. The water permeability of the membrane was 7200 $g/m^2/24$ h.

Example 12

Example 4 was repeated except that the ceric salt was replaced by 20 mM of $FeSO_4$ and the dose rate cut into half so that the total dose absorbed by the sample was 1800 Gy (180 krad). The resulted membrane had 41% graft. The water permeability of the membrane so produced was 7900 $g/m^2/24$ h and the water absorbance about 30% of the total film weight.

Example 13

Example 11 was repeated but the $CuSO_4$ was replaced by 20 mM $Fe_2(SO_4)_3$ and the solution was made

0.2 N in $H_2SO_4$. The resulted membrane had 21% graft. The water permeability of the membrane was 7200 $g/m^2/24$ h and the water absorption about 20% of the total film weight.

Example 14

Example 13 was repeated except that the concentration of $Fe_2(SO_4)_3$ was increased to 50 mM and the acidity was made 0.5 in $H_2SO_4$. The resulted membrane had 22% graft. The water permeability was 5700 $g/m^2/24$ h and the water absorption about 20% of the total film weight.

Example 15

Example 14 was repeated except that the $Fe_2(SO_4)_3$ was replaced by 50 mM $Ti_2(SO_4)_3$ and the solution made 1 N in $H_2SO_4$. The resulted membrane had 26% graft. The water permeability was 6200 $g/m^2/24$ h and the water absorption about 30% of the total film weight.

Example 16

Example 15 was repeated except that the $Ti_2(SO_4)_3$ was replaced by 20 mM $Ti(SO_4)_2$ and the acidity decreased to 0.3 N in $H_2SO_4$. The resulted membrane had 37% graft. The water permeability was 6200 $g/m^2/24$ h and the water absorption about 40% of the total film weight.

Example 17

Example 4 was repeated except that the $Ce^{4+}$ salt was replaced by 20 mM of $VOCl_2$. The resulted membrane had 86% graft. The water permeability of the membrane was 6700 $g/m^2/24$ h and the water absorption about 80% of the total film weight.

Example 18

Example 17 was repeated except that the $VOCl_2$ was replaced by 20 mM of $VCl_3$. The resulted membrane had 77% graft. The water permeability measured was 7600 $g/m^2/24$ h and the water absorption about 90% of the total film weight.

Example 19

Example 10 was repeated except that the 20% AAm was replaced by 20% HEMA and the $Cu(NO_3)_2$ concentration increased to 200 mM. The resulted membrane having a graft of 70% had water permeability of 2,400 $g/m^2/24$ h and water absorption of about 40% of the total film weight.

Example 20

Example 19 was repeated except that the solution was bubbled with $N_2$ for 30 min. prior to radiation and kept under nitrogen atmosphere during irradiation. The resulted membrane having a graft of 117% had water permeability of 3000 $g/m^2/24$ h and water absorption of about 30% of the total film weight.

Example 21

Example 19 was repeated except that the HEMA was replaced by 20% HEA and the $Cu(NO_3)_2$ decreased to 20 mM. The resulted membrane had a graft of 64% poly-HEA.

Example 22

Example 20 was repeated except that the HEMA was replaced by 10% AA. The $Cu(NO_3)_2$ concentration decreased to 20 mM and the irradiation dose increased to 5400 Gy (540 krad). The resulted membrane contained 32% graft. The acid form of the graft sheet was not easy to handle when dry because of self-adherence. In immersing the membrane into diluted $Na_2CO_3$ solution and the acid groups were neutralized and the self-adherence disappeared.

Example 23

Example 1 was repeated except that the ratio between the polyester and the diisocyanate in the polyurethane used was 3.8 (n in the formula given in example 1), and the modulus of elasticity and elongation at break of the polyurethane was 352 $N/cm^2$ (510 PSI) and 910% respectively. The resulted membrane contained 62% graft. The water permeability of the membrane so produced was 7300 $g/m^2/24h$, and the water absorption about 80% of the total film weight.

Example 24

Example 1 was repeated except that the ratio between the polyester and the diisocyanate in the polyurethane was 2.6 and the modulus of elasticity was 661 $N/cm^2$ (960 PSI) and the elongation at break was 580%. The irradiation time was increased to 150 min. so that the total dose absorbed by the sample was 3000 Gy (300 krad). The resulted membrane contained 44% graft. The water permeability of the membrane was 5600 $g/m^2/24$ h and the water absorption about 30% of the total film weight.

Example 25

Example 24 was repeated except that the polyurethane used had modulus of elasticity of 1626 $N/cm^2$

(2360 PSI) and elongation at break of 360%. The resulted membrane contained 18% graft. The membrane thus obtained had hydrophobic surface, insufficient elasticity and was not suitable for use as an artificial skin.

Examples 26—30

Sheet samples of polyester polyurethane, 30 μm thickness have been immersed in aqueous solution containing 20% AAm and 20 mM $Ce(NH_4)_2(NO_3)_6$ at room temp. (21±2°C) for different periods of time. Thereafter the sheets have been removed from the solution and washed thoroughly with warm water for several hours and the amount of graft was determined. The results of these experiments are given in Table I.

TABLE I:
Percent of graft on polyurethane in 20% AAm aqueous solution containing 20 mM $Ce(NH_4)_2(NO_3)_6$ at 21°C, without gamma irradiation.

| Example No. | Reaction time, hours | % Graft |
|---|---|---|
| 26 | 6 | 15 |
| 27 | 10 | 37 |
| 28 | 12 | 45 |
| 29 | 14 | 87 |
| 30 | 20 | 385 |

Changes in tensile properties of polyurethane films grafted with different percentages of AAm in dry and wet states were measured under controlled RH conditions and are given in Table II.

TABLE II
Tensile properties of AAm grafted polyurethane membranes as a function of percent of graft, in the dry and wet states, measured at a strain rate of 2 cm/min. and 25°C.

| Percent graft | Elongation at break % | | Tensile strength at break N/cm²(PSI) | | Modulus at 100% Elongation N/cm²(PSI) | |
|---|---|---|---|---|---|---|
| | dry* | wet** | dry* | wet** | dry* | wet** |
| 55 | 385 | 1070 | 4017 (5830) | 1261 (1830) | 1922 (2790) | 124 (180) |
| 62 | 330 | 950 | 4230 (6140) | 1192 (1730) | 2081 (3020) | 145 (210) |
| 73 | 325 | 880 | 4258 (6180) | 1144 (1950) | 2184 (3170) | 152 (220) |
| 88 | 295 | 630 | 4303 (6245) | 1578 (2290) | 2156 (3420) | 193 (280) |

* Measured at 50% RH
**Soaked in water and measured at 96% RH.

**Claims**

1. An artificial skin as a synthetic wound covering consisting of a thin, tough, elastic hydrophilic membrane whose thickness does not exceed 60 μm, having a water permeability of 2,400—8,000 g/m²/24h at 37°C and 60% relative humidity being a product obtained by grafting a hydrophilic monomer to a thin body of a polyester polyurethane having a recurring unit of the formula

$$-\!\!\!+(OR_1COOR_2CO)_nOR_1OCONH—C_6H_4CH_2C_6H_4—NHCO+\!\!\!-$$

in which $R_1$ and $R_2$ are each a group $—(CH_2)_x—$ where x is an integer of 1 to 8, and n has an average value of 2.5 to 20, which polyester polyurethane has a modulus of elasticity below 1034 $N/cm^2$ (1,500 PSI) and elongation at break above 500 %.

2. An artificial skin according to claim 1, wherein average n is from 2.5 to 8.

3. An artificial skin according to claim 1 or 2, wherein $R_1$ and $R_2$ are each a tetramethylene group $—(CH_2)_4—$.

4. An artificial skin according to any one of claims 1 to 3, wherein the hydrophilic monomer is a vinyl type monomer.

5. An artificial skin according to claim 4, wherein the hydrophilic monomer is acrylamide.

6. An artificial skin according to claim 4, wherein the hydrophilic monomer is hydroxyethyl acrylate.

7. An artificial skin according to claim 4, wherein the hydrophilic monomer is acrylic acid.

8. An artificial skin according to claim 4, wherein the hydrophilic monomer is hydroxyethyl methacrylate.

9. An artificial skin according to any one of claims 1 to 8 whose thickness is not less than 10 μm.

10. A method of making an artificial skin according to any one of claims 1 to 9 comprising preparing an aqueous solution of hydrophilic monomer and a salt of a transition metal whose ion is capable of serving as inhibitor for homopolymerization of the monomer, immersing into such a solution a film of a polyurethane as defined in any one of claims 1 to 3 and whose thickness does not exceed 60 μm, subjecting the solution with the film in it to conditions inducive of radical graft copolymerization and continuing the reaction until a product of the desired water permeability is obtained.

11. A process according to claim 10, wherein the induction of radical graft polymerization is effected by means of irradiation with an ionizing irradiation.

12. A process according to claim 11, wherein $^{60}Co$ is used as a source of said radiation.

13. A process according to any one of claims 10 to 12, wherein the inhibitor is a $Ce^{4+}$ salt.

14. A process according to claim 10, wherein the induction of radical graft copolymerization and simultaneous inhibition of homopolymerization is effected by the incorporation in solution of a $Ce^{4+}$ salt.

**Patentansprüche**

1. Künstliche Haut als synthetische Wundauflage, bestehend aus einer dünnen, zähen, elastischen hydrophilen Membran, deren Dicke 60 μm nicht überschreitet und die eine Wasserpermeabilität von 2400—8000 $g/m^2.24$ h bei 37°C und 60% relativer Feuchtigkeit besitzt und die ein Produkt ist, das erhalten worden ist durch Aufpfropfen eines hydrophilen Monomers auf einen dünnen Körper aus einem Polyesterpolyurethan mit einer wiederkehrenden Einheit der Formel

$$-\!\!\!+\!(OR_1COOR_2CO)_nOR_1OCONH—C_6H_4CH_2C_6H_4—NHCO\!+\!\!\!-$$

in der $R_1$ und $R_2$ jeweils eine Gruppe $—(CH_2)_x—$ bedeutet, wobei x eine ganze Zahl von 1 bis 8 ist und n einen mittleren Wert von 2,5—20 hat, wobei das Polyesterpolyurethan einen Elastizitätsmodul unterhalb 1034 $N/cm^2$ (1500 psi) und eine Bruchdehnung oberhalb 500% aufweist.

2. Künstliche Haute nach Anspruch 1, wobei der Mittelwert von n 2,5—8 beträgt.

3. Künstliche Haute nach Anspruch 1 oder 2, wobei $R_1$ und $R_2$ jeweils eine Tetramethylengruppe $—(CH_2)_4—$ ist.

4. Künstliche Haut nach einem der Ansprüche 1 bis 3, wobei das hydrophile Monomer ein Monomer vom Vinyltyp ist.

5. Künstliche Haut nach Anspruch 4, wobei das hydrophile Monomer Acrylamid ist.

6. Künstliche Haut nach Anspruch 4, wobei das hydrophile Monomer Hydroxyethyl-acrylat ist.

7. Künstliche Haut nach Anspruch 4, wobei das hydrophile Monomer Acrylsäure ist.

8. Künstliche Haut nach Anspruch 4, wobei das hydrophile Monomer Hydroxyethyl-methacrylat ist.

9. Künstliche Haut nach einem der Ansprüche 1 bis 8, dessen Dicke nicht weniger als 10 μm beträgt.

10. Verfahren zur Herstellung einer künstlichen Haut nach einem der Ansprüche 1 bis 9, umfassend die Herstellung einer wäßrigen Lösung von hydrophilem Monomer und einem Salz eines Übergangsmetalls, dessen Ion im Stande ist, als Inhibitor für die Homopolymerisation des Monomers zu dienen, Eintauchen eines Films aus einem Polyurethan, wie in einem der Ansprüche 1 bis 3 definiert und dessen Dicke nicht über 60 μm liegt, in eine solche Lösung, wobei die Lösung mit dem Film darin solchen Bedingungen ausgesetzt wird, die zu einer radikalischen Pfropfpolymerisation führen, und Fortführung der Reaktion bis ein Produkt mit der gewünschten Wasserpermeabilität erhalten wird.

11. Verfahren nach Anspruch 10, wobei die Einleitung der radikalischen Pfropfpolymerisation erreicht wird durch Bestrahlung mit ionisierender Strahlung.

12. Verfahren nach Anspruch 11, wobei $^{60}Co$ als Quelle für die Strahlung verwendet wird.

13. Verfahren nach einem der Ansprüche 10 bis 12, wobei der Inhibitor ein $Ce^{4+}$ Salz ist.

14. Verfahren nach Anspruch 10, wobei das Einleiten der radikalischen Pfropfpolymerisation und die gleichzeitige Hemmung der Homopolymerisation erreicht werden durch Zusatz eines $Ce^{4+}$ Salzes zu der Lösung.

**Revendications**

1. Peau artificielle à titre de recouvrement synthétique de plaies comprenant une membrane hydrophile mince, tenace et élastique dont l'épaisseur ne dépasse pas 60 µm, ayant une perméabilité à l'eau de 2400 à 8000 $g/m^2/24h$ à 37°C et 60% d'humidité relative, produit qu'on obtient par greffage d'un monomère hydrophile sur un corps mince d'un polyester-polyuréthane présentant un motif récurrent de formule:

$$-\!\!\!+(OR_1COOR_2CO)_nOR_1OCONH—C_6H_4CH_2H_4—NHCO+\!\!\!-$$

dans laquelle $R_1$ et $R_2$ représentent chacun le groupe $—(CH_2)_x—$ dans lequel x est un nombre entier de 1 à 8 et *n* a une valeur moyenne de 2,5 à 20, ce polyester-polyuréthane ayant un module d'élasticité inférieur à 1034 $N/cm^2$ (1500 psi) et un allongement à la rupture de plus de 500%.

2. Peau artificielle selon la revendication 1, dans laquelle la valeur moyenne de *n* est de 2,5 à 8.

3. Peau artificielle selon la revendication 1 ou 2, dans laquelle $R_1$ et $R_2$ représentant chacun un groupe tétraméthylène $—(CH_2)_4—$.

4. Peau artificielle selon l'une quelconque des revendications 1 à 3 dans laquelle le monomère hydrophile est un monomère du type vinyle.

5. Peau artificielle selon la revendication 4, dans laquelle le monomère hydrophile est l'acrylamide.

6. Peau artificielle selon la revendication 4, dans laquelle le monomère hydrophile est l'acrylate d'hydroxyéthyle.

7. Peau artificielle selon la revendication 4, dans laquelle le monomère hydrophile est l'acide acrylique.

8. Peau artificielle selon la revendication 4, dans laquelle le monomère hydrophile est le méthacrylate d'hydroxyéthyle.

9. Peau artificielle selon l'une quelconque des revendications 1 à 8, dont l'épaisseur n'est pas inférieure à 10 µm.

10. Procédé de fabrication d'une peau artificielle selon l'une quelconque des revendications 1 à 9 qui comprend les étapes consistant à préparer une solution aqueuse d'un monomère hydrophile et d'un sel de métal de transition dont l'ion est capable de servir d'inhibiteur à l'homopolymérisation du monomère, à immerger dans une telle solution une pellicule d'un polyuréthane tel que défini dans l'une quelconque des revendications 1 à 3 et dont l'épaisseur ne dépasse pas 60 µm, à soumettre la solution contenant la pellicule à des conditions qui produisent une copolymérisation radicalaire de greffage et à poursuivre la réaction jusqu'à obtenir un produit ayant la perméabilité désirée à l'eau.

11. Procédé selon la revendication 10, dans lequel on effectue l'induction de la polymérisation radicalaire de greffage par une irradiation ionisante.

12. Procédé selon la revendication 11, dans lequel on utilise comme source de ladite radiation le $^{60}Co$.

13. Procédé selon l'une quelconque des revendications 10 à 12, dans lequel l'inhibiteur est un sel de $Ce^{4+}$.

14. Procédé selon la revendication 10, dans lequel on effectue l'induction de la copolymérisation radicalaire de greffage et l'inhibition simultanée de l'homopolymérisation par incorporation dans la solution d'un sel de $Ce^{4+}$.